# EUROPÄISCHE PATENTSCHRIFT

(11) **EP 4 257 232 B1**
(45) Veröffentlichungstag und Bekanntmachung des Hinweises auf die Patenterteilung: **28.08.2024**
(21) Anmeldenummer: 22166434.5
(22) Anmeldetag: 04.04.2022
(51) Int. Cl.: B01F 33/501, B01F 31/441, B01F 35/71, B01F 35/75, B01F 35/32

(54) **VORRICHTUNG UND VERFAHREN ZUM BEREITSTELLEN VON KNOCHENZEMENTTEIG**
DEVICE AND METHOD FOR PRODUCING BONE CEMENT
DISPOSITIF ET PROCÉDÉ DE PRÉPARATION DE LA PÂTE DE CIMENT OSSEUX

(43) Veröffentlichungstag der Anmeldung: 11.10.2023
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- EP-A1- 0 692 229
- EP-A1- 3 320 869

## Beschreibung

Die Erfindung betrifft eine Vorrichtung zum Bereitstellen eines Knochenzementteigs aus zwei Ausgangskomponenten, umfassend eine hohlzylinderförmige Kartusche mit einem Innenraum, wobei in einem
proximalen Teil des Innenraums ein Knochenzementpulver als erste Ausgangskomponente und in einem distalen Teil des Innenraums mindestens ein Beutel enthaltend eine Monomerflüssigkeit als zweite Ausgangskomponente lagert,
wobei der Innenraum am proximalen Teil des Innenraums über einen Vakuumanschluss mit einer Unterdruckquelle verbindbar ist,
wobei zwischen dem Knochenzementpulver und dem mindestens einen Beutel ein axial im Innenraum beweglicher Austragskolben und auf einer dem Austragskolben axial gegenüberliegenden Seite des mindestens einen Beutel ein axial im Innenraum (210) beweglicher Förderkolben angeordnet ist, so dass bei einem Zusammenschieben von Austragskolben und Förderkolben der mindestens eine Beutel fluidleitend geöffnet werden und die Monomerflüssigkeit in den distalen Teil des Innenraums fließen kann,
wobei der proximale Teil und der distale Teil des Innenraums über ein Leitungsmittel fluidleitend miteinander verbunden sind.

Die Erfindung betrifft weiterhin ein Verfahren zum Bereitstellen eines Knochenzementteigs mit einer derartigen Vorrichtung.

### Hintergrund der Erfindung

Es werden erhebliche Bemühungen unternommen, Vorrichtungen und Verfahren zum Bereitstellen von Knochenzement aufzuzeigen, mittels derer Knochenzementteig einfach, zuverlässig und schnell bereitgestellt werden kann. Ein wichtiger Aspekt bei der Bereitstellung von Knochenzementteig ist die Vermeidung von Lufteinschlüssen, wie beispielsweise Gasblasen, im Knochenzement. Zu deren Vermeidung wurden eine Vielzahl von Vakuum-Zementiersystemen beschreiben, von denen exemplarisch folgende genannt sind: US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzementteig. Eine Weiterentwicklung besteht in der Entwicklung von Zementiersystemen, in denen beide Ausgangskomponenten in separaten Bereichen der Mischsysteme gelagert sind und erst unmittelbar vor der Zementierapplikation im Zementiersystem miteinander vermischt werden. Solche geschlossenen, sogenannten Full-Prepacked-Systeme, sind in folgenden Schriften genannt: EP 0 692 229 A1, DE 10 2009 031 178 B3, US 5,997,544 A, US 6,709,149 B1, DE 698 12 726 T2, EP 0 796 653 A2, US 5,588,745 A.

In der europäischen Patentschrift EP 0 692 229 B1 wird eine Vorrichtung beschrieben, in der ein mit Monomerflüssigkeit gefüllter Beutel axial hinter einem mit einem Knochenzementpulver gefülltem Bereich innerhalb einer Kartusche lagert. Zwischen Knochenzementpulver und Beutel ist ein Austragskolben angeordnet. Auf der dem Austragskolben axial gegenüberliegenden Seite des Beutels ist ein Förderkolben angeordnet. Um einen Knochenzementteig bereitzustellen, werden Austragskolben und Förderkolben zusammengeschoben, wodurch es zu einem fluidleitenden Öffnen des Beutels kommt und in Folge die Monomerflüssigkeit aus dem Beutel austritt. Durch fortgesetztes Zusammenschieben der beiden Kolben wird der Beutel zusammengepresst und die Monomerflüssigkeit durch ein Leitungsmittel im Austragskolben in das Knochenzementpulver gefördert. Durch Vermischen der geförderten Monomerflüssigkeit und dem Knochenzementpulver wird der Knochenzementteig bereitgestellt.

Nachteilig gestaltet sich an einer derartigen Vorrichtung, dass zum im Wesentlichen vollständigen Fördern der Monomerflüssigkeit in das Knochenzementpulver der Beutel im Wesentlichen vollständig zusammengepresst werden muss. Aufgrund der stabilen und recht starren Wandung solch handelsüblicher Beutel erfordert dies eine erhebliche Kraftanstrengung durch den Anwender der Vorrichtung, was die Verletzungsgefahr bei der Verwendung der Vorrichtung erhöht. Weiterhin ist ein im Wesentlichen vollständiges Zusammenpressen derartiger Beutel nicht möglich, so dass immer ein Anteil der Monomerflüssigkeit im Beutel verbleiben wird. Zudem kann es beim Zusammenpressen des Beutels zur Ausbildung von fluidleitend geschlossenen, mit Monomerflüssigkeit gefüllten Faltungen in der Wandung des Beutels kommen, welche das im Wesentlichen vollständige Fördern der Monomerflüssigkeit in das Knochenzementpulver verhindern.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, eine Vorrichtung zur Verfügung zu stellen, welche ein im Wesentlichen vollständiges Fördern von Monomerflüssigkeit aus einem Beutel in ein Knochenzementpulver erlaubt und gleichzeitig den dafür benötigten Kraftaufwand für den Anwender der Vorrichtung möglichst minimiert.

Es ist eine weitere Aufgabe der Erfindung, ein Verfahren bereitzustellen, mit dem Knochenzement aus zwei Ausgangskomponenten bereitgestellt werden kann, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist eine Vorrichtung zum Bereitstellen eines Knochenzementteigs aus zwei Ausgangskomponenten, umfassend
eine hohlzylinderförmige Kartusche mit einem Innenraum, wobei in einem proximalen Teil des Innenraums ein Knochenzementpulver als erste Ausgangskomponente und in einem distalen Teil des Innenraums mindestens ein Beutel enthaltend eine Monomerflüssigkeit als zweite Ausgangskomponente lagert,
wobei der Innenraum am proximalen Teil des Innenraums über einen Vakuumanschluss mit einer Unterdruckquelle verbindbar ist,
wobei zwischen dem Knochenzementpulver und dem mindestens einen Beutel ein axial im Innenraum beweglicher Austragskolben und auf einer dem Austragskolben axial gegenüberliegenden Seite des mindestens einen Beutels ein axial im Innenraum beweglicher Förderkolben angeordnet ist, so dass bei einem Zusammenschieben von Austragskolben und Förderkolben der mindestens eine Beutel fluidleitend geöffnet werden und die Monomerflüssigkeit in den distalen Teil des Innenraums fließen kann,
wobei der proximale Teil und der distale Teil des Innenraums über ein Leitungsmittel fluidleitend miteinander verbunden sind,
dadurch gekennzeichnet, dass
sich das Leitungsmittel vom Austragskolben in den distalen Teil des Innenraums mit einer axialen Leitungsmittellänge erstreckt, welche mindestens einem Viertel einer Beutellänge des Beutels entspricht, so dass der Austragskolben und der Förderkolben beim Zusammenschieben durch das Leitungsmittel mit einem Kolbenabstand beabstandet sind, welcher mindestens einem Viertel der Beutellänge entspricht.

In einer Ausführungsform der Vorrichtung ist das Leitungsmittel im Wesentlichen formstabil. Diese Ausführungsform ist eine zweite Ausführungsform der Erfindung, welche vorzugsweise von der ersten Ausführungsform der Erfindung abhängt.

In einer Ausführungsform der Vorrichtung ist das Leitungsmittel zumindest abschnittsweise als Hohlzylinder mit mindestens einer axial in einer Hohlzylinderwand verlaufenden fluidleitenden Zylinderdurchführung ausgeformt. Diese Ausführungsform ist eine dritte Ausführungsform der Erfindung, welche vorzugsweise von der ersten oder zweiten Ausführungsform der Erfindung abhängt.

In einer Ausführungsform der Vorrichtung weist der Hohlzylinder einen Hohlzylinderinnendurchmesser auf, welcher eine zumindest teilweise Aufnahme des mindestens einen Beutels erlaubt. Diese Ausführungsform ist eine vierte Ausführungsform der Erfindung, welche bevorzugt von der dritten Ausführungsform der Erfindung abhängt.

In einer Ausführungsform der Vorrichtung ist das Leitungsmittel zumindest abschnittsweise als ein Rohr, als ein Schlauch oder als ein Rohr und ein Schlauch ausgeformt. Diese Ausführungsform ist eine fünfte Ausführungsform der Erfindung, welche vorzugsweise von einer der vorhergehenden Ausführungsformen der Erfindung, insbesondere von der ersten oder zweiten Ausführungsform der Erfindung, abhängt.

In einer Ausführungsform der Vorrichtung weist das Leitungsmittel einen Leitungsmittelinnendurchmesser von 0,5 mm bis 2 mm auf. Diese Ausführungsform ist eine sechste Ausführungsform der Erfindung, welche vorzugsweise von einer der vorhergehenden Ausführungsformen der Erfindung abhängt.

In einer Ausführungsform der Vorrichtung ist im distalen Teil des Innenraums mindestens ein Öffnungsmittel zum Öffnen des mindestens einen Beutels angeordnet. Diese Ausführungsform ist eine siebte Ausführungsform der Erfindung, welche vorzugsweise von einer der vorhergehenden Ausführungsformen der Erfindung abhängt.

In einer Ausführungsform der Vorrichtung ist das Öffnungsmittel als Dorn ausgeformt, weist das Öffnungsmittel mindestens eine Schneidkante auf oder ist als Dorn ausgeformt und weist mindestens eine Schneidkante auf. Diese Ausführungsform ist eine achte Ausführungsform der Erfindung, welche vorzugweise von der siebten Ausführungsform der Erfindung abhängt.

In einer Ausführungsform der Vorrichtung ist ein dem Austragskolben axial gegenüberliegendem proximalen Innenraumende des proximalen Teils des Innenraums mit einem Kartuschenkopf fluidleitend verschlossen. Diese Ausführungsform ist eine neunte Ausführungsform der Erfindung, welche vorzugsweise von einer der vorhergehenden Ausführungsformen der Erfindung abhängt.

In einer Ausführungsform der Vorrichtung ist der Vakuumanschluss am Kartuschenkopf angeordnet. Diese Ausführungsform ist eine zehnte Ausführungsform der Erfindung, welche vorzugsweise von der neunten Ausführungsform der Erfindung abhängt.

In einer Ausführungsform der Vorrichtung ist in einer Kartuschenkopfdurchführung des Kartuschenkopfs ein axial im proximalen Teil des Innenraums verschiebbares Mischrohr angeordnet. Diese Ausführungsform ist eine elfte Ausführungsform der Erfindung, welche vorzugsweise von der neunten oder zehnten Ausführungsform der Erfindung abhängt.

In einer Ausführungsform der Vorrichtung ist innerhalb des Mischrohrs ein lösbarer Mischstab angeordnet. Diese Ausführungsform ist eine zwölfte Ausführungsform der Erfindung, welche vorzugsweise von der elften Ausführungsform der Erfindung abhängt.

Eine dreizehnte Ausführungsform der Erfindung ist ein Verfahren zum Bereitstellen eines Knochenzementteigs aus zwei Ausgangskomponenten mittels einer Vorrichtung nach einer der vorhergehenden Ausführungsformen der Erfindung, umfassend die folgenden Schritte:
a. Zusammenschieben von Austragskolben und Förderkolben unter Öffnen des mindestens einen Beutels;
b. Fortgesetztes Zusammenschieben von Förderkolben und Austragskolben bis das Leitungsmittel am Förderkolben angeordnet ist;
c. Fördern der Monomerflüssigkeit durch das Leitungsmittels in den proximalen Teil des Innenraums durch Anlegen eines Unterdrucks am Vakuumanschluss.

In einer Ausführungsform des Verfahrens unter Verwendung einer Vorrichtung nach der zwölften Ausführungsform der Erfindung, wird in einem Schritt d. das Knochenzementpulver und die Monomerflüssigkeit im proximalen Teil des Innenraums mittels des Mischstabs bei angelegtem Unterdruck unter Bereitstellung des Knochenzementteigs vermischt. Diese Ausführungsform ist eine vierzehnte Ausführungsform der Erfindung, welche vorzugweise von der dreizehnten Ausführungsform der Erfindung abhängt.

In einer Ausführungsform des Verfahrens wird in einem Schritt e. der Mischstab vom Mischrohr gelöst, der Mischstab aus dem Mischrohr gezogen und der angemischte Knochenzementteig aus dem proximalen Teil des Innenraums durch das Mischrohr aus der Vorrichtung durch Vortreiben des Austragskolbens in Richtung des Kartuschenkopfs ausgetragen. Diese Ausführungsform der Erfindung ist eine fünfzehnte Ausführungsform der Erfindung, welche vorzugsweise von der vierzehnten Ausführungsform der Erfindung abhängt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B. Ein "im Wesentlichen vollständiges Fördern einer Komponente C" umfasst beispielweise ein Fördern von ≥90 bis ≤100 Volumen-%, insbesondere ≥95 bis ≤100 Volumen-%, des Gesamtvolumens von C.

Die Begriffe "proximal" und "distal" dienen lediglich der Bezeichnung der räumlich entgegengesetzten Enden der Vorrichtung oder anderer Struktureinheiten der Vorrichtung und erlauben keinen Rückschluss auf die Orientierung in Bezug zu einem menschlichen Körper, beispielsweise eines Anwenders der Vorrichtung. "Distal zu ..." und "proximal zu..." oder ähnliche Formulierungen drücken entsprechend lediglich die räumliche Anordnung zweier Struktureinheiten der Vorrichtung zueinander aus.

### Ausführliche Beschreibung

Ein erster Gegenstand der Erfindung betrifft eine Vorrichtung zum Bereitstellen eines Knochenzementteigs aus zwei Ausgangskomponenten, umfassend
eine hohlzylinderförmige Kartusche mit einem Innenraum, wobei in einem
proximalen Teil des Innenraums ein Knochenzementpulver als erste Ausgangskomponente und in einem distalen Teil des Innenraums mindestens ein Beutel enthaltend eine Monomerflüssigkeit als zweite Ausgangskomponente lagert,
wobei der Innenraum am proximalen Teil des Innenraums über einen Vakuumanschluss mit einer Unterdruckquelle verbindbar ist,
wobei zwischen dem Knochenzementpulver und dem mindestens einen Beutel ein axial im Innenraum beweglicher Austragskolben und auf einer dem Austragskolben axial gegenüberliegenden Seite des mindestens einen Beutels ein axial im Innenraum beweglicher Förderkolben angeordnet ist, so dass bei einem Zusammenschieben von Austragskolben und Förderkolben der mindestens eine Beutel fluidleitend geöffnet werden und die Monomerflüssigkeit in den distalen Teil des Innenraums fließen kann,
wobei der proximale Teil und der distale Teil des Innenraums über ein Leitungsmittel fluidleitend miteinander verbunden sind,
dadurch gekennzeichnet, dass
sich das Leitungsmittel vom Austragskolben in den distalen Teil des Innenraums mit einer axialen Leitungsmittellänge erstreckt, welche mindestens einem Viertel einer Beutellänge des Beutels entspricht, so dass der Austragskolben und der Förderkolben beim Zusammenschieben durch das Leitungsmittel mit einem Kolbenabstand beabstandet sind, welcher mindestens einem Viertel der Beutellänge entspricht.

Die Vorrichtung weist ein Leitungsmittel auf, durch welches der distale Teil und der proximale Teil des Innenraums fluidleitend miteinander verbunden sind. Fluidleitend bedeutet, dass der distale Teil und der proximale Teil des Innenraums für Flüssigkeiten, insbesondere die Monomerflüssigkeit, und für Gase durchlässig verbunden sind.

Das Leitungsmittel erstreckt sich innerhalb des distalen Teils des Innenraums ausgehend vom Austragskolben in Richtung des Förderkolbens, wobei es innerhalb des distalen Teils des Innenraums eine axiale Leitungsmittellänge aufweist, welche mindestens einem Viertel einer Beutellänge des Beutels entspricht. Vorzugsweise sind das Leitungsmittel und der mindestens eine Beutel zumindest abschnittsweise nebeneinander im distalen Teil des Innenraums angeordnet, so dass die Vorrichtung möglichst kompakt ausgeformt ist. Das Leitungsmittel erstreckt sich weiterhin durch den Austragskolben hindurch oder am Austragskolben vorbei, um den distalen Teil des Innenraums fluidleitend mit dem proximalen Teil des Innenraums zu verbinden. Dabei kann der sich in den distalen Teil des Innenraums erstreckende Abschnitt des Leitungsmittels und der durch den Austragskolben hindurch oder am Austragskolben vorbei verlaufende Abschnitt des Leitungsmittels einteilig oder mehrteilig ausgestaltet sein.

Da der mindestens eine Beutel aus sterischen Gründen vorzugsweise mit seiner Längsachse parallel zur Längsachse der Vorrichtung gelagert ist und der Abstand von Austragskolben und Förderkolben im Ruhezustand der Vorrichtung vorzugsweise im Wesentlichen der Beutellänge entspricht, entspricht die axiale Leitungsmittellänge im Ruhezustand vorzugsweise im Wesentlichen mindestens einem Viertel des Abstands von Austragskolben zu Förderkolben. Beim Zusammenschieben von Austragskolben und Förderkolben, wobei vorzugsweise der Austragskolben in Richtung des Förderkolbens verschoben wird und der Förderkolben vorzugsweise im Wesentlichen an seinem ursprünglichen Platz verbleibt, wird der zumindest eine Beutel, beispielsweise durch Druckausübung auf den Beutel, fluidleitend geöffnet und die Monomerflüssigkeit tritt in den distalen Teil des Innenraums aus. Dabei können der Austragskolben und der Förderkolben so weit aufeinander zubewegt werden, bis das Leitungsmittel mit einem dem Förderkolben zugewandten Leitungsmittelende am Förderkolben angeordnet ist, vorzugsweise den Förderkolben berührt, und ein fortgesetztes Zusammenschieben nicht weiter möglich ist, ohne die strukturelle Integrität des Leitungsmittels zu zerstören. Das Leitungsmittel beabstandet somit den Austragskolben und den Förderkolben mit einem Kolbenabstand, welcher mindestens einem Viertel der Beutellänge entspricht.

Das Leitungsmittel sorgt somit nach dem Zusammenschieben von Austragskolben und Förderkolben für einen Kolbenabstand, welcher zumindest einem Viertel der Beutellänge entspricht, und erlaubt gleichzeitig ein im Wesentlichen vollständiges Fördern der in Folge des Austretens aus dem Beutel an den Förderkolben angrenzender Monomerflüssigkeit in den proximalen Teil des Innenraums. Das Leitungsmittel ist somit so ausgestaltet, dass ein im Wesentlichen vollständiges Fördern der Monomerflüssigkeit aus dem zumindest einen Beutel in den proximalen Teil des Innenraums möglich ist, ohne dass der zumindest eine Beutel im Wesentlichen vollständig zusammengepresst werden muss und dabei der Austragskolben und der Förderkolben im Wesentlichen vollständig, lediglich beabstandet durch den zusammengepressten Beutel, zusammengeschoben werden müssen. Dies erleichtert dem Anwender der Vorrichtung die Verwendung der Vorrichtung, da ein im Wesentlichen vollständiges Zusammenpressen des zumindest einen Beutels einen erheblichen Kraftaufwand seitens des Anwenders bedarf. Zudem besteht die Gefahr, dass sich beim im Wesentlichen vollständigen Zusammenpressen des zumindest einen Beutels mit Monomerflüssigkeit gefüllt Hohlräume im Beutel bilden, so dass die Monomerflüssigkeit nicht im Wesentlichen vollständig aus dem Beutel ausfließen kann und ein im Wesentlichen vollständiges Fördern der Monomerflüssigkeit in den proximalen Teil des Innenraums unterbunden wird.

Es ist vorteilhaft, die Monomerflüssigkeit nach dem Ausfließen aus dem fluidleitend geöffneten Beutel angrenzend an den Förderkolben zu sammeln und von dort aus in den proximalen Teil des Innenraums zu fördern, anstatt die Monomerflüssigkeit angrenzend an den Austragskolben zu sammeln und von dort, beispielsweise durch eine fluidleitende Durchführung durch den Austragskolben, in den proximalen Teil des Innenraums zu fördern. Letzteres birgt ein Risiko eines unkontrollierten und/oder ungewollten Fließens der Monomerflüssigkeit in den proximalen Teil des Innenraums, was ein unkontrolliertes und/oder ungewolltes Ausbilden eines Knochenzementteigs zur Folge hätte. Es ist bevorzugt, dass die Vorrichtung neben dem Leitungsmittel keine weiteren Mittel, wie beispielsweise zusätzliche Durchführungen durch den Austragskolben, aufweist, welche den distalen Teil mit dem proximalen Teil des Innenraums fluidleitend verbinden.

Um ein zuverlässiges fluidleitendes Öffnen des zumindest einen Beutels durch das Zusammenschieben von Austragskolben und Förderkolben zu gewährleisten, entspricht die axiale Leitungsmittellänge und/oder der Kolbenabstand vorzugsweise maximal einem Dreiviertel der Beutellänge.

In einer Ausgestaltungsform sind der Austragskolben und das Leitungsmittel einteilig ausgestaltet.

Die Monomerflüssigkeit wird vorzugsweise durch das Leitungsmittel gefördert, indem über den Vakuumanschluss mittels einer Unterdruckquelle, beispielsweise einer Vakuumpumpe, ein Unterdruck im proximalen Teil des Innenraums angelegt wird, wodurch die Monomerflüssigkeit durch das Leitungsmittel aus dem distalen Teil des Innenraums gesaugt wird.

Um zu verhindern, dass Knochenzementpulver aus dem proximalen Teil in den distalen Teil des Innenraums und die mindestens zwei Beutel, insbesondere Beutelteilstücke, aus dem distalen Teil in den proximalen Teil des Innenraums über das Leitungsmittel gelangen können, ist das Leitungsmittel vorzugsweise mit einem Filtermittel, insbesondere einer Porenscheibe, beispielsweise aus gesinterten Polypropylenpartikeln, aus gesinterten oder verpressten Polyethylenfasern, aus Cellulosefilz oder aus Pappe, ausgestattet, welche das Leitungsmittel für Feststoffe undurchlässig ausgestaltet.

Innerhalb des distalen Teils des Innenraums der Vorrichtung lagert mindestens ein, bevorzugt ein bis drei, weiter bevorzugt ein bis zwei, Beutel enthaltend eine Monomerflüssigkeit. Unter einem Beutel wird eine nicht-starre, weitestgehend flexible Lagermöglichkeit verstanden, welche die Monomerflüssigkeit hermetisch dicht und steril lagern kann und mittels Druckausübung oder Einwirkung eines Öffnungsmittels, beispielsweise durch Aufstechen, Aufschneiden oder Aufreißen, zu öffnen ist. Die Beutel können beispielsweise aus einer Mehrschichtverbundfolie, vorzugsweise aufweisend eine EVOH-Sperrschicht gefertigt sein. Optional können die Beutel eine Metallbeschichtung, insbesondere eine Aluminiumbeschichtung, aufweisen.

Die Vorrichtung weist eine hohlzylinderförmige Kartusche auf. Unter einer hohlzylinderförmigen Kartusche ist ein rohrartiges Behältnis zu verstehen, welches einen Innenraum und eine den Innenraum umgebende Kartuschenwand aufweist. Der Querschnitt der Kartusche kann beliebige Formen annehmen. Aufgrund der einfachen Fertigung und der anwendungssichereren Verwendung der Vorrichtung ist der Querschnitt, und bevorzugt auch der Querschnitt des Innenraums, kreisförmig ausgestaltet. Dies erlaubt eine gute Handhabbarkeit für den Anwender und reduziert durch eine Abwesenheit von Kanten ein Risiko einer Verkeilung beweglicher Teile innerhalb der Vorrichtung. Erfindungsgemäß kann die Kartusche aus unterschiedlichsten Materialien oder Materialkombinationen bestehen. Beispiele kann die Vorrichtung aus einem Polymer bestehen. Bevorzugt handelt es sich bei dem Polymer um ein transparentes Polymer, da der Anwender auf diese Weise eine ordnungsgemäße Funktion der Vorrichtung während einer Verwendung optisch kontrollieren kann.

Zwischen dem Knochenzementpulver und dem Beutel ist ein axial im Innenraum beweglicher Austragskolben angeordnet. Der Austragskolben dient der räumlichen Trennung des mindestens einen Beutels und dem Knochenzementpulver, so dass weder Knochenzementpulver vom proximalen Teil des Innenraums in den distalen Teil des Innenraums noch der mindestens eine Beutel vom distalen Teil des Innenraums in den proximalen Teil des Innenraums gelangen können. Insbesondere letzteres verhindert einen mit Beutel oder Beutelteilstücken durchsetzten Knochenzementteig, welcher gesundheitliche Risiken für den Patienten darstellen und ein ordnungsgemäßes Austragen des Knochenzementteigs aus der Vorrichtung beeinträchtigen könnte. Der Austragskolben unterteilt den Innenraum in den proximalen und den distalen Teil des Innenraums. Der Austragskolben dient bevorzugt weiterhin dem Austragen des bereitgestellten Knochenzementteigs aus der Vorrichtung. Dazu kann der Austragskolben aus seiner ursprünglichen Position axial in Richtung des bereitgestellten Knochenzementteigs verschoben werden.

Die Vorrichtung weist einen axial im Innenraum beweglichen Förderkolben auf. Der Förderkolben ist auf der dem Austragskolben axial gegenüberliegenden Seite des mindestens einen Beutels innerhalb der Vorrichtung angeordnet. Durch eine Relativbewegung von Förderkolben und Austragskolbens zueinander, welche den Abstand von Förderkolben und Austragskolben innerhalb des Innenraums verkürzt, beispielsweise durch ein Verschieben des Förderkolbens in Richtung des Austragskolbens oder bevorzugt durch ein Verschieben des Austragskolbens in Richtung des Förderkolbens, kommt es zu einem Öffnen des mindestens einen Beutels, beispielsweise durch ein Aufplatzen des Beutels durch Druckausübung durch die zwei Kolben.

Um ein ungewolltes Zusammenschieben von Austragskolbens und Förderkolben zu verhindern, kann am Austragskolben und/oder am Förderkolben ein Rastmittel angeordnet sein, so dass der Austragskolben und/oder der Förderkolben mit der Kartusche, insbesondere mit der Kartuschenwand, rasten kann. Bevorzugt wird zum Öffnen des mindestens einen Beutels der Austragskolben in Richtung des Förderkolbens verschoben, da dies im proximalen Teil des Innenraums mehr Platz lässt, um den Knochenzementteig aus den Ausgangskomponenten gründlich zu durchmischen. Daher ist es bevorzugt, dass insbesondere der Förderkolben nicht unbeabsichtigt aus seiner ursprünglichen Position verschiebbar ist, insbesondere durch Kräfte, welche beim Öffnen des mindestens einen Beutels auf den Förderkolben einwirken.

Dabei wird durch das Rastmittel erreicht, dass zunächst der mindestens eine Beutel durch Vortreiben des Austragskolbens geöffnet werden kann und die daraus austretende Monomerflüssigkeit über das Leitungsmittel in den proximalen Teil des Innenraums der Kartusche, also in das Knochenzementpulver, gepresst und/oder gesaugt werden kann, wobei der Förderkolben dabei relativ zur Kartusche und zum Innenraum seine ursprüngliche Position hält. Erst nachdem die Monomerflüssigkeit weitgehend in das Knochenzementpulver gepresst und/oder gesaugt wurde, und somit der Knochenzementteig im proximalen Teil des Innenraums der Kartusche vorliegt, kann anschließend der Knochenzementteig mit dem Förderkolben aus dem proximalen Teil der Kartusche gedrückt werden. Die Kraft zur Lösung der Rastung ist also größer als die zur Öffnung des mindestens einen Beutels und dem Fördern der Monomerflüssigkeit über das Leitungsmittel in den proximalen Teil des Innenraums notwendige Kraft.

In einer Ausführungsform kann das Leitungsmittel durch das Zusammenschieben von Austragskolben und Förderkolben in seiner axialen Leitungsmittellänge verkürzt werden, beispielsweise indem das Leitungsmittel gestaucht wird oder radial zur Längsachse der Vorrichtung gebogen oder geknickt wird. In dieser Ausführungsform kann der durch das Leitungsmittel bewirkte Kolbenabstand kürzer als die axiale Leitungsmittellänge ausfallen. Vorzugsweise ist das Leitungsmittel so ausgestaltet, dass der durch das Leitungsmittel bewirkte Kolbenabstand zumindest 90% der axialen Leitungsmittellänge entspricht.

In einer bevorzugten Ausführungsform ist das Leitungsmittel im Wesentlichen formstabil, so dass der durch das Leitungsmittel bewirkte Kolbenabstand der axialen Leitungsmittellänge entspricht. Die axiale Leitungsmittellänge wird durch das Zusammenschieben von Austragskolben und Förderkolben im Wesentlichen nicht verändert.

Das Leitungsmittel kann unterschiedlich ausgeformt sein, um den Austragskolben und den Förderkolben mit dem Kolbenabstand zu beabstanden und gleichzeitig ein Fördern der Monomerflüssigkeit in den proximalen Teil des Innenraums zu gewährleisten.

Eine Ausführungsform der Vorrichtung ist dadurch gekennzeichnet, dass das Leitungsmittel zumindest abschnittsweise als Hohlzylinder, vorzugsweise als durchgängiger Hohlzylinder, mit mindestens einer axial in einer Hohlzylinderwand verlaufenden fluidleitenden Zylinderdurchführung ausgeformt ist. Die Hohlzylinderwand sorgt dabei für die Beabstandung des Förderkolbens und des Austragskolbens mit dem Kolbenabstand und die Zylinderdurchführung innerhalb der Hohlzylinderwand, welche vorzugsweise an einem dem Förderkolben zugewandten Hohlzylinderende mündet, erlaubt das Fördern der Monomerflüssigkeit in den proximalen Teil des Innenraums. Um ein Fördern der Monomerflüssigkeit möglichst unabhängig von der räumlichen Ausrichtung der Vorrichtung zu gewährleisten, ist es bevorzugt, dass der Hohlzylinder mindestens drei, bevorzugt mindestens vier, weiter bevorzugt mindestens sechs Zylinderdurchführungen aufweist. Bevorzugt weist der Hohlzylinder maximal zehn Zylinderdurchführungen. Bevorzugt sind die Zylinderdurchführungen gleichmäßig aber den Hohlzylinder verteilt.

In einer Ausführungsform ist der Hohlzylinder aus getrennten Hohlzylindersegmenten, insbesondere in axialer Richtung verlaufenden getrennten Hohlzylindersegmenten, ausgebildet, wobei es bevorzugt ist, dass in jedem der Hohlzylindersegmente zumindest eine Zylinderdurchführung ausgebildet ist. Beispielsweise kann der Hohlzylinder in zwei bis acht, bevorzugt in vier bis sechs, Hohlzylindersegmente unterteilt sind.

Um die Vorrichtung möglichst kompakt und anwendungssicher auszugestalten, ist eine Ausführungsform der Vorrichtung dadurch gekennzeichnet, dass der Hohlzylinder einen Hohlzylinderinnendurchmesser aufweist, welcher eine zumindest teilweise Aufnahme des mindestens einen Beutels erlaubt. Vorzugsweise entspricht der Hohlzylinderinnendurchmesser mindestens einer Beutelbreite des Beutels, so dass der mindestens eine Beutel, und bei Anwesenheit mehrerer Beutel alle Beutel, mit dem radialen Umfang zumindest abschnittsweise innerhalb des Hohlzylinders gelagert werden kann beziehungsweise können. Dadurch kann der oder die Beutel in den Hohlzylinder "eingeschoben" gelagert werden. Dies erlaubt eine Platzsparende Anordnung des mindestens einen Beutels innerhalb des distalen Teil des Innenraums. Vorzugsweise verläuft die Hohlzylinderwand im Wesentlichen direkt angrenzend an der Kartuschenwand, was eine kompakte Ausgestaltung der Vorrichtung erlaubt.

Eine Ausführungsform der Vorrichtung ist dadurch gekennzeichnet, dass das Leitungsmittel zumindest abschnittsweise als Rohr, als Schlauch oder als Rohr und als Schlauch ausgeformt ist. Dies erlaubt eine einfache Herstellung sowie leichte und platzsparende Anordnung des Leitungsmittels innerhalb des distalen Teil des Innenraums. Dabei ist die Ausformung als Rohr bevorzugt, da ein Rohr formstabiler ist und so sicherer eine Beabstandung des Austragskolbens und Förderkolbens durch das Leitungsmittel ermöglicht. Das Leitungsmittel kann aus einem oder mehreren Rohren und/oder Schläuchen ausgeformt werden. Vorzugsweise umfasst das Leitungsmittel ein bis sechs, vorzugsweise ein bis vier Rohre und/oder Schläuche.

Um ein möglichst schnelles Fördern der Monomerflüssigkeit durch das Leitungsmittel in den proximalen Teil des Innenraums zu erlauben, ist eine Ausführungsform der Vorrichtung dadurch gekennzeichnet, dass das Leitungsmittel einen fluidleitenden Leitungsmittelinnendurchmesser in einem Bereich von 0,5 mm bis 2 mm, bevorzugt in einem Bereich von 0,5 mm bis 1,5 mm aufweist. Kleinere Durchmesser verlangsamen das Fördern der Monomerflüssigkeit. Größere Durchmesser erschweren aufgrund geringerer Kapillareffekte im Leitungsmittel ein im Wesentlichen vollständiges Fördern der Monomerflüssigkeit.

Der mindestens eine Beutel kann beispielsweise beim Zusammenschieben von Austragskolben und Förderkolben durch eine Druckausübung, welche durch die beiden Kolben auf den Beutel ausgeübt wird, fluidleitend geöffnet werden.

Um ein fluidleitendes Öffnen des Beutels durch ein Zusammenschieben von Austragskolben und Förderkolben zu erleichtern, ist eine Ausführungsform der Vorrichtung dadurch gekennzeichnet, dass im distalen Teil des Innenraums mindestens ein Öffnungsmittel zum Öffnen des Beutels angeordnet ist.

Unter einem Öffnungsmittel ist eine ausgeformte Struktureinheit oder Strukturuntereinheit zu verstehen, welche einen Beutel öffnen, insbesondere aufstechen, aufschneiden oder aufreißen, kann, wenn das Öffnungsmittel und der Beutel gegeneinander geschoben werden. Um das Öffnen, insbesondere Aufstecken, Aufschneiden oder Aufreißen, zu erleichtern, ist es bevorzugt, dass die Öffnungsmittel mit einer im Vergleich zu einer Querschnittsfläche des Innenraums geringen Fläche gegen die Beutel pressbar sind, die Öffnungsmittel also im weitestgehenden Sinne "scharf und/oder "spitz" sind.

Je nach Beschaffenheit des Beutels und des Öffnungsmittels kann zum Öffnen des Beutels durch das Öffnungsmittel ein unterschiedlich hoher Krafteintrag notwendig sein. Insbesondere ist der Krafteintrag durch die Öffnungsmittel, im Vergleich zu einem benötigten Krafteintrag durch Öffnen des mindestens einen Beutels mittels zweier planarer Kolben, verringert.

In einer Ausgestaltungsform ist mindestens ein Öffnungsmittel am Austragskolben, angeordnet. In einer weiteren Ausgestaltungsform ist mindestens ein Öffnungsmittel am Förderkolben angeordnet. In einer weiteren, bevorzugten Ausgestaltungsform ist mindestens ein Öffnungsmittel am Austragskolben und mindestens ein Öffnungsmittel am Förderkolben angeordnet, was ein fluidleitendes Öffnen des mindestens einen Beutel bereits bei geringfügigem Zusammenschieben von Austragskolben und Förderkolben erleichtert.

In einer Ausführungsform ist das Leitungsmittel als Öffnungsmittel ausgeformt. Beispielsweise kann das Leitungsmittel als Rohr ausgeformt sein, wobei das Leitungsmittel an dem dem Förderkolben zugewandten Ende spitz zulaufend, beispielsweise als Dorn, ausgeformt ist. Das Leitungsmittel erleichtert somit das fluidleitende Öffnen des mindestens einen Beutels.

Eine Ausführungsform der Vorrichtung ist dadurch gekennzeichnet, dass das Öffnungsmittel als Dorn ausgeformt ist. Eine weitere Ausführungsform ist dadurch gekennzeichnet, dass das Öffnungsmittel mindestens eine Schneidkante aufweist. Eine weitere Ausführungsform der Vorrichtung ist dadurch gekennzeichnet, dass das Öffnungsmittel als Dorn ausgeformt ist und mindestens eine Schneidkante aufweist.

Um ein unbeabsichtigtes Öffnen des mindestens einen Beutels, beispielsweise beim Transport der Vorrichtung, zu verhindern, kann es bevorzugt sein, dass im distalen Teil des Innenraums zwischen dem Öffnungsmittel und dem mindestens einen Beutel eine Transportsicherung angeordnet ist. Die Transportsicherung kann beispielsweise aus einer Platte mit Perforationen für den späteren Durchtritt der Öffnungsmittel gebildet sein, die sich mittels bei Krafteinwirkung einknickenden Stegen an der distalen Austragskolbenseite beabstandet.

Eine Ausführungsform der Vorrichtung ist dadurch gekennzeichnet, dass ein dem Austragskolben axial gegenüberliegendes proximales Innenraumende des proximalen Teils des Innenraums mit einem Kartuschenkopf fluidleitend verschlossen ist. Der Kartuschenkopf ist vorzugweise reversibel, beispielsweise mittels einer Gewindeverbindung, mit der Kartusche verbindbar und erlaubt ein einfaches Befüllen des proximalen Teils des Innenraums mit dem Knochenzementpulver. In einer Ausgestaltungsform sind die Kartusche und der Kartuschenkopf einteilig ausgestaltet.

Eine Ausführungsform der Vorrichtung ist dadurch gekennzeichnet, dass der Vakuumanschluss am Kartuschenkopf angeordnet ist. Dies ermöglicht eine einfache Herstellungsweise der Vorrichtung und verortet den Vakuumanschluss gleichzeitig räumlich möglichst weit entfernt vom Austragskolben und insbesondere vom Leitungsmittel, so dass nicht unbeabsichtigt die durch das Leitungsmittel in den proximalen Teil des Innenraums geförderte Monomerflüssigkeit direkt über den Vakuumanschluss in die mit der Vorrichtung verbindbare Unterdruckquelle gefördert wird.

Der in der Vorrichtung bereitgestellte Knochenzementteig kann auf unterschiedliche Weise aus der Vorrichtung ausgebracht werden.

Eine Ausführungsform der Vorrichtung ist dadurch gekennzeichnet, dass im Kartuschenkopf eine fluidleitende Kartuschenkopfdurchführung angeordnet ist, durch welche der in der Vorrichtung, insbesondere im proximalen Teil des Innenraums mittels des Knochenzementpulvers und der Monomerflüssigkeit, bereitgestellte Knochenzementteig austragbar ist. Vorzugsweise kann dazu der Austragskolben in Richtung des Kartuschenkopfs verschoben werden, was ein Auspressen des Knochenzementteigs aus der Vorrichtung bewirkt. Bevorzugt wird dabei der Austragskolben durch ein Vorschieben des Förderkolbens in Richtung des Kartuschenkopfs bewegt. Dabei übt der Förderkolben eine Kraft auf das Leitungsmittel auf, welche diese auf den Austragskolben überträgt.

Zum Anmischen der beiden Ausgangskomponenten unter Bereitstellung des Knochenzementteigs ist eine Ausführungsform der Vorrichtung dadurch gekennzeichnet, dass in der Kartuschenkopfdurchführung ein axial im proximalen Teil des Innenraums verschiebbares fluidleitendes Mischrohr angeordnet ist. In einer Ausgestaltungsform kann durch das Mischrohr von außerhalb der Vorrichtung ein Mischelement, beispielsweise in Form eines Rührers, in den proximalen Teil des Innenraums eingebracht werden kann, um die Ausgangskomponenten nach erfolgtem Fördern der Monomerflüssigkeit durch das Leitungsmittel in den proximalen Teil des Innenraums zu durchmischen. Beispielsweise kann ein Mischelement so weit in das Mischrohr eingeschoben werden, dass das Mischelement aus dem Mischrohr in den proximalen Teil des Innenraums herausragt und durch ein wiederholtes axiales Auf- und Abbewegen des Mischrohrs mit dem Mischelement die Ausgangskomponenten vermischt werden. In einer bevorzugten Ausgestaltungsform weist das Mischrohr selbst ein Mischelement auf, um die Ausgangskomponenten durch wiederholtes axiales Auf- und Abbewegen des Mischrohrs zu durchmischen. Vorzugsweise ist dabei an einem dem Austragskolben zugewandten Mischrohrende eine Mischscheibe angeordnet.

Nach erfolgter Durchmischung der Ausgangskomponenten, und bei Bedarf nach dem Entfernen des Mischstabs aus dem Mischrohr, kann das Mischrohr als Austragsschnorchel zur kontrollierten Abgabe des Knochenzementteigs aus der Vorrichtung verwendet werden. Dazu wird das Mischrohr vorzugsweise möglichst weit axial aus dem Kartuschenkopf herausgezogen und dann mit dem Kartuschenkopf fixiert, beispielsweise mittels eines Schraubgewindes.

Eine Ausführungsform der Vorrichtung ist dadurch gekennzeichnet, dass innerhalb des Mischrohrs ein lösbarer Mischstab zum verbesserten Mischen der Ausgangskomponenten innerhalb des proximalen Teils des Innenraums angeordnet ist. Der Mischstab kann ein Mischelement darstellen, welches sich durch axial das Mischrohr erstreckt und zum Mischen der Ausgangskomponenten fest mit dem Mischrohr verbunden ist. Der Mischstab weist dazu an einem dem Austragskolben zugewandten Mischstabende vorzugsweise ein Mischelement auf. Weist das Mischrohr bereits selbst ein Mischelement auf, dient der Mischstab dem erleichterten Bedienen des Mischrohrs. Zudem verbessert der Mischstab Handhabung der Vorrichtung und vermindert die Gefahr eines Abknickens des Mischrohrs beim Anmischen der Ausgangskomponenten. Weiterhin verschließt der Mischstab das Mischrohr fluidleitend und verhindert so, dass sich innerhalb des Mischrohrs die Ausgangskomponenten ansammeln, welche innerhalb des Mischstabs einer gründlichen Durchmischung durch das Mischrohr nicht zugänglich sind.

An einem dem Austragskolben entgegengesetztem Mischstabende weist der Mischstab vorzugsweise einen Griff auf, um den Mischstab mitsamt dem damit verbundenen Mischrohr reversibel axial im proximalen Teil des Innenraums auf und ab zubewegen, um die Ausgangskomponenten zu durchmischen.

Vorzugsweise ist der Mischstab über den Griff mit dem Mischrohr lösbar verbunden, so dass durch Entfernen des Griffs der Mischstab aus dem Mischrohr herausziehbar ist. Dadurch kann nach der Bereitstellung des Knochenzementteigs das Mischrohr als Austragsschnorchel für den Knochenzementteig verwendet werden. Bevorzugt wird dazu das Mischrohr möglichst weit aus der Vorrichtung herausgezogen und anschließend fest mit dem Kartuschenkopf verbunden, so dass das Mischrohr nicht mehr axial beweglich ist. Dies kann vorzugsweise über ein Schraubgewinde geschehen.

Ein weiterer Gegenstand der Erfindung betrifft Verfahren zum Bereitstellen eines Knochenzementteigs aus zwei Ausgangskomponenten mittels einer Ausführungsform der vorbeschriebenen Vorrichtung, umfassend die folgenden Schritte:
a. Zusammenschieben von Austragskolben und Förderkolben unter Öffnen des mindestens einen Beutels;
b. Fortgesetztes Zusammenschieben von Förderkolben und Austragskolben bis das Leitungsmittel am Förderkolben angeordnet ist;
c. Fördern der Monomerflüssigkeit durch das Leitungsmittels in den proximalen Teil des Innenraums durch Anlegen eines Unterdrucks am Vakuumanschluss.

Durch das Zusammenschieben von Austragskolben und Förderkolben kommt es zu einem fluidleitenden Öffnen des mindestens einen Beutels, so dass die im Beutel enthaltende Monomerflüssigkeit in den distalen Teil des Innenraums ausfließt. Vorzugsweise erfolgt das Zusammenschieben von Austragskolben und Förderkolben derart, dass der Austragskolben in Richtung des Förderkolbens verschoben wird, während der Förderkolben im Wesentlichen an seiner ursprünglichen Position innerhalb der Kartusche verbleibt. Dies sorgt für eine räumliche Vergrößerung des proximalen Teils des Innenraums, so dass ein nachfolgendes Anmischen des Knochenzementteigs aus den beiden Ausgangskomponenten erleichtert ist. Um den mindestens einen Beutel einfacher durch das Zusammenschieben der beiden Kolben öffnen zu können, ist es bevorzugt, dass das Öffnen mittels einer Öffnungsmittels, wie beispielsweise einem Dorn an mindestens einem der beiden Kolben, unterstützt wird.

Nach dem Öffnen des mindestens einen Beutels werden der Austragskolben und der Förderkolben weiter zusammengeschoben, bis das Leitungsmittel am Förderkolben angeordnet ist, insbesondere bis das Leitungsmittel in Kontakt mit dem Förderkolben kommt, und die beiden Kolben somit durch das Leitungsmittel beabstandet sind. Das Leitungsmittel sorgt dabei dafür, dass die Monomerflüssigkeit vom an dem räumlich an den Förderkolben angrenzenden Bereich des distalen Teils des Innenraums in den proximalen Teil des Innenraums gefördert werden kann. Dabei braucht der mindestens eine Beutel aufgrund der Ausgestaltung des Leitungsmittel im Wesentlichen nicht vollständig zusammengepresst werden, was sowohl die Anwendung der Vorrichtung für den Anwender aufgrund eines geringeren Krafteinsatzes erleichtert, als auch die Gefahr von Monomerflüssigkeiteinschlüssen innerhalb eines ansonsten zusammengepressten Beutels verringert.

Ein Anteil der aus dem mindestens einen Beutel ausgetretenen Monomerflüssigkeit kann bereits durch das fortgesetzte Zusammenschieben von Förderkolben und Austragskolben nach dem Öffnen des Beutels in den proximalen Teil des Innenraums gefördert werden. Um ein im Wesentlichen vollständiges Fördern der Monomerflüssigkeit aus dem distalen Teil in den proximalen Teil des Innenraums zu erreichen, wird, vorzugsweise erst nachdem das Leitungsmittel am Förderkolben angeordnet ist, am Vakuumanschluss ein Unterdruck angelegt, beispielsweise durch Verbinden des Vakuumanschlusses mit einer Vakuumpumpe.

Eine Ausführungsform des Verfahrens ist dadurch gekennzeichnet, dass nach dem im Wesentlichen vollständigen Fördern der Monomerflüssigkeit in den proximalen Teil des Innenraums über das Leitungsmittel das Knochenzementpulver und die Monomerflüssigkeit im proximalen Teil des Innenraums mittels eines Mischstabs bei angelegtem Unterdruck unter Bereitstellung des Knochenzementteigs vermischt werden. Vorzugsweise wird der Mischstab von außerhalb der Vorrichtung betätigt. Das Anmischen bei angelegtem Unterdruck vermindert Gaseinschlüsse im bereitgestellten Knochenzementteig, was zu einer verbesserten mechanischen Festigkeit des aus dem Knochenzementteig durch Aushärtung erzeugten Knochenzement beiträgt.

Der bereitgestellte Knochenzementteig kann auf unterschiedliche Weise aus der Vorrichtung ausgebracht werden. Beispielsweise kann der Knochenzementteig mit einem Spatel aus dem proximalen Innenraum herausgeholt werden.

Eine Ausführungsform des Verfahrens ist dadurch gekennzeichnet, dass nach dem Anmischen des Knochenzementteigs der dazu verwendete Mischstab vom Mischrohr der Vorrichtung gelöst und aus diesem durch Ziehen entfernt wird, so dass der angemischte Knochenzementteig aus dem proximalen Teil des Innenraums durch das Mischrohr aus der Vorrichtung durch Vortreiben des Austragskolbens in Richtung des Kartuschenkopfs ausgetragen wird. Vorzugsweise erfolgt das Vortreiben des Austragskolbens durch ein Vortreiben des Förderkolbens, welcher über das Leitungsmittel auf den Austragskolben einwirkt. Dies reduziert die zum Austragen des Knochenzementteigs notwendigen Arbeitsschritte des Anwenders und die dazu notwendigen Werkzeuge.

Vorzugsweise wird zum Austragen des Knochenzementteigs aus der Vorrichtung die Vorrichtung mit einer Austragshilfe, insbesondere einer Austragspistole, verbunden, welche den Förderkolben, und damit auch den Austragskolben, in Richtung des Kartuschenkopfs verschiebt und so den Knochenzementteig aus dem proximalen Teil des Innenraums auspresst.

Die Vorrichtung ist dadurch gekennzeichnet, dass diese einen Knochenzementteig aus zwei Ausgangskomponenten bereitstellt. Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Durch Aushärtung wird aus einem Knochenzementteig ein Knochenzement. Bevorzugt handelt es sich bei diesen Knochenzementen um Polymethylmethacrylat-Knochenzemente (PMMA Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

Die für die Vorrichtung offenbarten Merkmale sind auch für das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: einen schematischen Längsschnitt einer Vorrichtung zum Bereitstellen eines Knochenzementteigs umfassend zwei Beutel enthaltend eine Monomerflüssigkeit und ein Leitungsmittel in Form eines Hohlzylinders,
- Fig. 2: die Vorrichtung aus Figur 1 mit fluidleitend geöffneten Beuteln,
- Fig. 3: die Vorrichtung aus den Figuren 1 und 2, wobei ein Austragskolben und ein Förderkolben der Vorrichtung durch das Leitungsmittel beabstandet sind,
- Fig. 4: die Vorrichtung aus den Figuren 1 bis 3, wobei die Monomerflüssigkeit im Wesentlichen vollständig durch das Leitungsmittel gefördert wurde,
- Fig. 5: die Vorrichtung aus den Figuren 1 bis 4 mit bereitgestelltem Knochenzementteig,
- Fig. 6: die Vorrichtung aus den Figuren 1 bis 5 bereit zum Austragen des bereitgestellten Knochenzementteigs,
- Fig. 7: die Vorrichtung aus den Figuren 1 bis 6 beim Austragen des bereitgestellten Knochenzementteigs,
- Fig. 8: einen schematischen Längsschnitt durch eine weitere Vorrichtung zum Bereitstellen eines Knochenzementteigs umfassend ein Leitungsmittel in Form von vier Rohren, und
- Fig. 9: ein Flussdiagramm eines Verfahrens zum Bereitstellen eines Knochenzementteigs.

### Beschreibung der Figuren

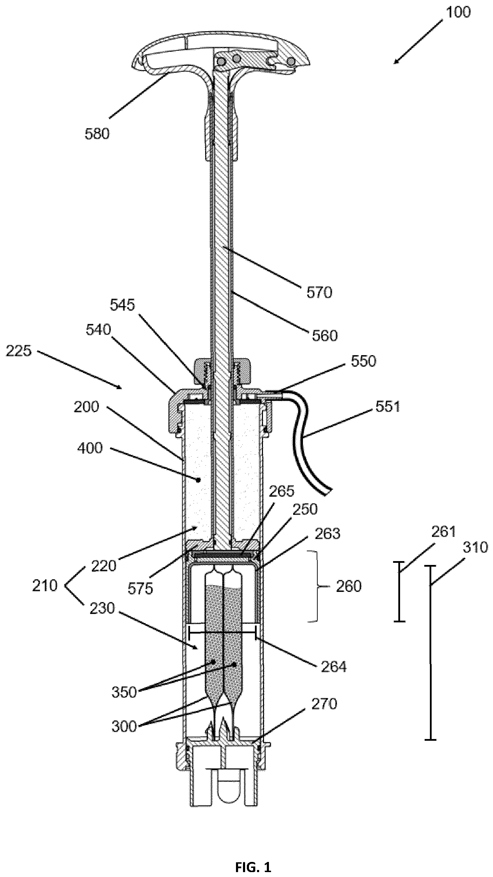

**Figur 1** zeigt einen schematischen Längsschnitt einer beispielhaften Ausführung einer Vorrichtung 100 zum Bereitstellen eines Knochenzementteigs in einem Ausgangszustand. Die Vorrichtung 100 ist einstückig, aber aus mehreren Bauteilen aufgebaut. Die Vorrichtung 100 ist rohrartig aufgebaut und umfasst eine hohlzylinderförmige Kartusche 200 mit einem Innenraum 210. In einem proximalen Teil 220 des Innenraums 210 ist ein Knochenzementpulver 400 als eine erste Ausgangskomponente und in einem distalen Teil 230 des Innenraums 210 sind zwei Beutel 300 enthaltend eine Monomerflüssigkeit 350 als zweite Ausgangskomponente des Knochenzementteigs gelagert.

Der proximale Teil 220 und der distale Teil 230 des Innenraums 210 sind durch einen axial im Innenraum 210 reversibel beweglichen Austragskolben 250 separiert. Der Austragskolben 250 ist für Feststoffe undurchlässig ausgestaltet, so dass kein Knochenzementpulver 400 vom proximalen Teil 210 in den distalen Teil 230 des Innenraums 210 und die Beutel 300 nicht vom distalen Teil 230 in den proximalen Teil 220 des Innenraums 210 gelangen. Dazu weist der Austragskolben 250 eine Porenscheibe 265 auf.

Der Austragskolben 250 weist ein Leitungsmittel 260 auf, welches den distalen Teil 230 mit dem proximalen Teil 220 des Innenraums fluidleitend verbindet. Das Leitungsmittel 260 erstreckt sich tunnelartig durch den Austragskolben und ist abschnittsweise als Hohlzylinder mit fluidleitenden Zylinderdurchführungen 263 (nur exemplarisch mit Bezugszeigen versehen) ausgeformt, welcher sich vom Austragskolben 250 distal in Richtung eines Förderkolbens 270 erstreckt, welcher dem Austragskolben auf der axial gegenüberliegenden Seite der Beutel 300 im Innenraum 210 angeordnet ist. Dabei weist das Leitungsmittel 260 eine axiale Leitungsmittellänge 261, welche ungefähr einem Drittel einer axialen Beutellänge 310 der Beutel 300 entspricht. Das Leitungsmittel 300 in Form eines Hohlzylinders weist einen Hohlzylinderinnendurchmesser 264 auf, welcher ein abschnittsweises axiales Einführen der Beutel 300 in den vom Hohlzylinder umfangenen Hohlraum erlaubt. Dies sorgt für eine möglichst kompakte Bauweise der Vorrichtung 100. In der gezeigten Ausführungsform sind das Leitungsmittel 260 und der Austragskolben 250 einteilig ausgestaltet.

Der Förderkolben 270 schließt ein distales Innenraumende fluidleitend ab, so dass die Monomerflüssigkeit 350 dort nicht unbeabsichtigt austreten kann. Der Förderkolben 270 ist reversibel axial im Innenraum 210 verschiebbar und ausgestaltet, um mit einer Austragspistole (nicht gezeigt) reversibel verbindbar zu sein, um einem Anwender das axiale Verschieben im Innenraum 210 zu erleichtern.

An einem proximalen Innenraumende 225 des proximalen Teils 220 des Innenraums 210 ist die Kartusche 200 mit einem Kartuschenkopf 540 verschlossen, so dass das Knochenzementpulver 400 nicht unbeabsichtigt aus dem Innenraum 210 austreten kann. Der Kartuschenkopf 540 weist mittig eine Kartuschenkopfdurchführung 545 auf, durch welche ein Mischrohr 560 axial beweglich in den proximalen Teil 220 des Innenraums 210 eingeführt ist. Innerhalb des Mischrohrs 560 ist ein Mischstab 570 angeordnet, welcher lösbar mit dem Mischrohr 560 über einen Griff 580 an einem proximalen Mischrohrende befestigt ist und so zusammen mit dem Mischrohr 560 axial im proximalen Teil 220 des Innenraums 210 bewegbar ist. Der Griff 580 dient der vereinfachten Handhabung der Vorrichtung 100 durch einen Anwender, indem er ein axiales Bewegen des Mischrohrs 560 samt Mischstab 570 innerhalb des Innenraums 210 erleichtert. An einem dem Austragskolben 250 zugewandten distalen Mischrohrende ist das Mischrohr 560 mit einer Mischscheibe 575 ausgestattet, welche ein Anmischen von Monomerflüssigkeit 350 und Knochenzementpulver 400 erleichtert.

Am Kartuschenkopf 540 ist ein Vakuumanschluss 550 angebracht, über welchen der proximale Teil 220 des Innenraums 210 fluidleitend mit einer Unterdruckquelle (nicht gezeigt) verbindbar ist. In der gezeigten Ausführungsform ist der Vakuumanschluss 550 als rohrartige Ausstülpung ausgeformt, welche den Innenraum 210 mittels eines Schlauch 551 mit der Unterdruckquelle verbindbar macht.

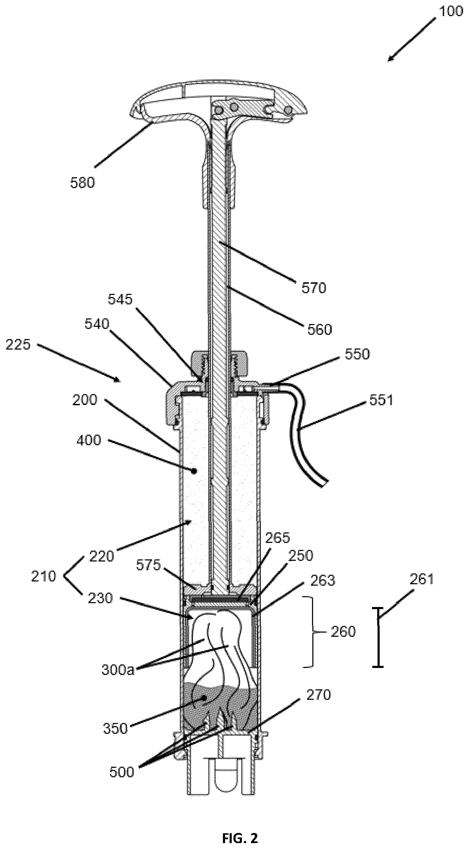

**Figur 2** zeigt den Längsschnitt der Vorrichtung 100 aus Figur 1, wobei in Figur 2 der Austragskolben 250 und der Förderkolben 270 im Vergleich zu Figur 1 teilweise zusammengeschoben sind. Um das Zusammenschieben von Austragskolben 250 und Förderkolben 270 zu erleichtern, kann dies durch eine Krafteinwirkung auf den Griff 580 in Richtung des Förderkolbens 270 geschehen. Der Griff 580 übertragt dabei die Krafteinwirkung über den Mischstab 570 auf den Austragskolben 250.

Durch das teilweise Zusammenschieben des Austragskolbens 250 und des Förderkolbens 270 wurden die Beutel 300 der Figur 1 gegen am Förderkolben 270 angeordnete Öffnungsmittel 500 in Form von Dornen gepresst, so dass diese in Figur 2 als fluidleitend geöffnete Beutel 300a vorliegen und die Monomerflüssigkeit 350 in den distalen Teil 230 des Innenraums 210 ausgeflossen ist.

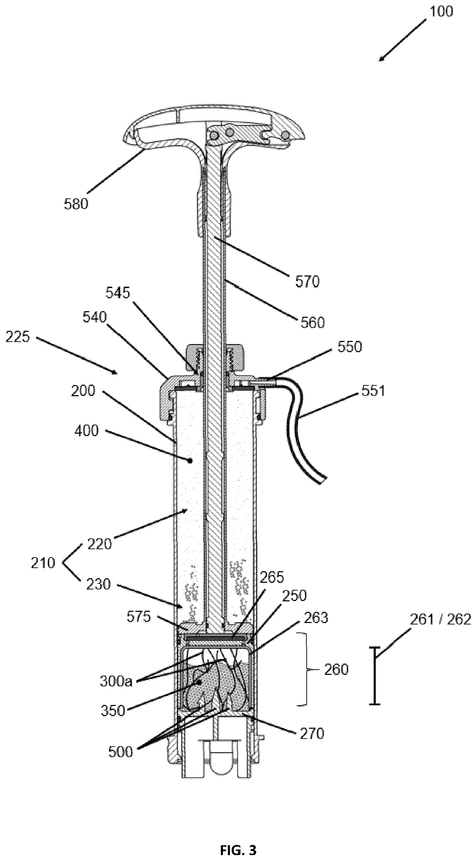

**Figur 3** zeigt den Längsschnitt der Vorrichtung 100 aus den Figuren 1 und 2, wobei in Figur 3 der Austragskolben 250 so weit in Richtung des Förderkolbens 270 verschoben ist, dass die beiden Kolben 250, 270 durch das Leitungsmittel 300 beabstandet sind. Das Leitungsmittel 260 ist in der gezeigten Vorrichtung 100 formstabil ausgeformt, so dass ein weitergehendes Zusammenschieben von Austragskolben 250 und Förderkolben 270 nicht möglich ist, ohne die strukturelle Integrität des Leitungsmittels 260 zu zerstören. Der Austragskolben 250 und der Förderkolben 270 weisen somit einen Kolbenabstand 262 zueinander auf, welcher im Wesentlichen der axialen Leitungsmittellänge 261 entspricht.

Durch das Zusammenschieben von Austragskolben 250 und Förderkolben 270 kann die Monomerflüssigkeit 350 bereits zu einem geringen Anteil in den proximalen Teil 220 des Innenraums 210 gefördert worden sein (angedeutet durch kleine Tröpfchen im proximalen Teil 220 des Innenraums 210 proximal zum Austragskolben 250). Die geöffneten Beutel 300a sind in der gezeigten Position des Austragskolben 250 zusammengeschoben, allerdings nicht in einem Maße, welche den Anwender der Vorrichtung 100 eine große Kraftanstrengung abnötigt.

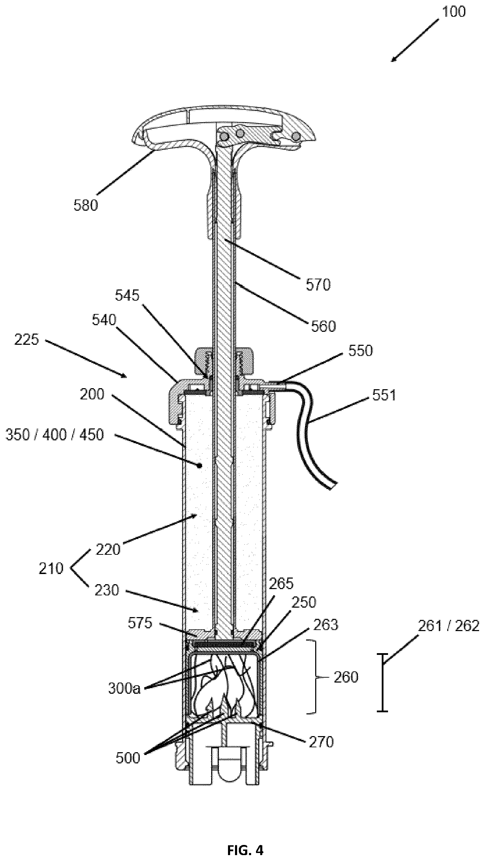

**Figur 4** zeigt den Längsschnitt der Vorrichtung 100 aus den Figuren 1 bis 3, wobei in Figur 4 die Monomerflüssigkeit 350 aus den Figuren 1 bis 3 im Wesentlichen vollständig in den proximalen Teil 220 des Innenraums 210 durch das Leitungsmittel 260, insbesondere die Zylinderdurchführungen 263 des Leitungsmittels 260, gefördert wurde. Im proximalen Teil 220 des Innenraums 210 liegen somit nun die Monomerflüssigkeit 350, das Knochenzementpulver 400 und bereits zu einem geringen Anteil aus der Monomerflüssigkeit 350 und dem Knochenzementpulver 400 durch Inkontaktbringen derselben ein Knochenzementteig 450 vor.

Um die Monomerflüssigkeit 350 aus dem distalen Teil 230 in den proximalen Teil 220 des Innenraums 210 durch das Leitungsmittel 260 zu fördern, wurde über den Vakuumanschluss 550 und den Schlauch 551 mittels einer Unterdruckquelle (nicht gezeigt) ein Unterdruck im proximalen Teil 220 des Innenraums 210 angelegt.

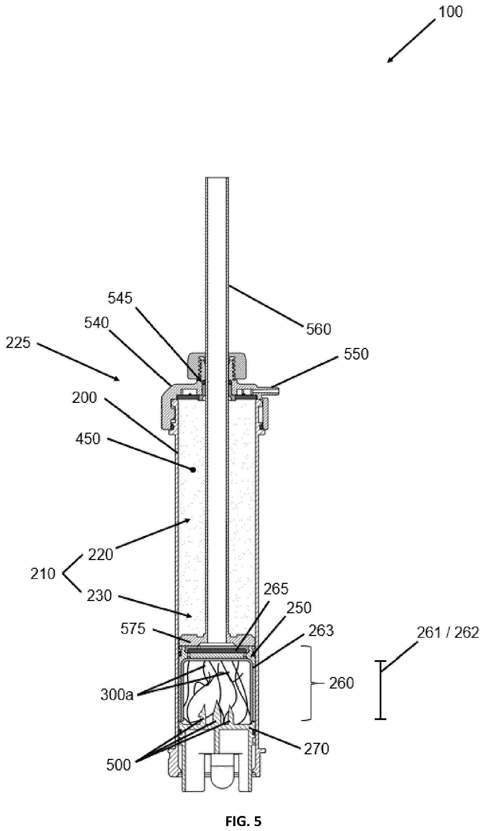

**Figur 5** zeigt den Längsschnitt der Vorrichtung 100 aus den Figuren 1 bis 4, wobei in Figur 5 die Monomerflüssigkeit 350 und das Knochenzementpulver 400 aus den vorherigen Figuren im Wesentlichen vollständig zu Knochenzementteig 450 umgesetzt sind. Dazu wurde das Mischrohr 560 samt Mischscheibe 575 wiederholt axial im proximalen Teil 220 des Innenraums 210 auf- und abbewegt (nicht gezeigt) und im Anschluss der lösbare Griff 580 samt Mischstab 570 aus dem Mischrohr 560 entfernt. Das Anmischen der Ausgangskomponenten findet vorzugweise unter Einfluss des Unterdrucks statt, welcher über den Vakuumanschluss 550 zum Fördern der Monomerflüssigkeit 350 in den proximalen Teil 220 des Innenraums bereitgestellt wurde. Nach dem Anmischen ist der Unterdruck nicht mehr vonnöten, so dass der Schlauch 551 entfernt wurde.

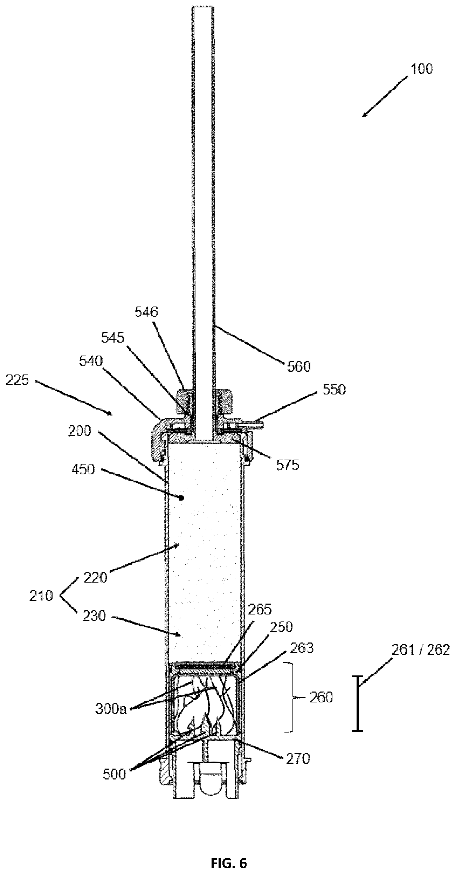

**Figur 6** zeigt den Längsschnitt der Vorrichtung 100 aus den Figuren 1 bis 5, wobei in Figur 6 das Mischrohr 560 so weit durch die Kartuschenkopfdurchführung 545 aus dem proximalen Teil 220 des Innenraums 210 herausgezogen ist, bis die Mischscheibe 575 distal am Kartuschenkopf 540 anliegt. Nach dem Entfernen des Mischstabs 570 aus dem Mischrohr 560 (vgl. Figur 5) kann das Mischrohr 560 somit als fluidleitender Austragsschnorchel für den bereitgestellten Knochenzementteig 450 dienen. Damit das Mischrohr 560 beim Austragen des Knochenzementteigs 450 nicht unbeabsichtigt wieder in den proximalen Teil 220 des Innenraums 210 eingeschoben wird, ist es mit einem Schraubgewinde 546 reversibel in der Kartuschenkopfdurchführung 545 fixiert.

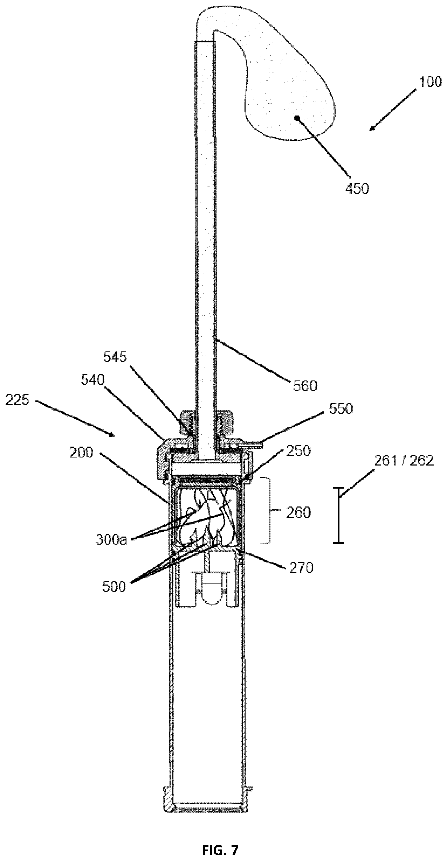

**Figur 7** zeigt den Längsschnitt der Vorrichtung 100 aus den Figuren 1 bis 6, wobei in Figur 7 der Förderkolben 270, und somit über das Leitungsmittel 260 auch der Austragskolben 250, in Richtung des Kartuschenkopfs 540 verschoben ist. Dadurch wurde der bereitgestellte Knochenzementteig 450 anteilig aus dem proximalen Teil 220 des Innenraums 210 über das Mischrohr 560 aus der Vorrichtung 100 ausgetragen. Um das Verschieben des Förderkolbens 270 für den Anwender der Vorrichtung 100 zu erleichtern, kann der Förderkolben 270 mit einer Austragshilfe, beispielsweise einer Austragspistole, verbunden sein (nicht gezeigt). Dazu ist der Förderkolben 270 an seiner dem Austragskolben 250 abgewandten Seite mit einem Förderkolbenanschluss ausgestattet, um den Förderkolben 270 reversibel mit der Austragshilfe zu verbinden.

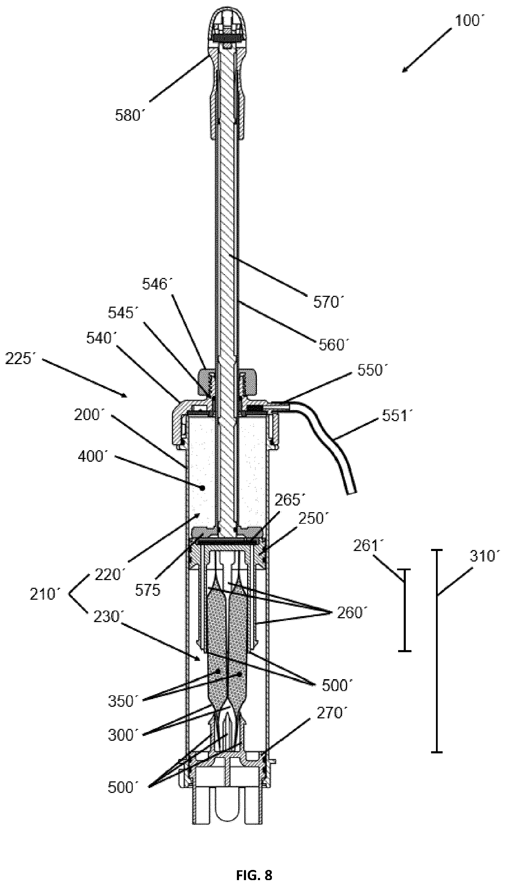

**Figur 8** zeigt einen schematischen Längsschnitt einer weiteren beispielhaften Ausführung einer Vorrichtung 100' zum Bereitstellen eines Knochenzementteigs in einem Ausgangszustand. Die Ausführungsform der Vorrichtung 100' stimmt weitgehend mit der vorstehend beschriebenen und in den Figuren 1 bis 7 dargestellten Ausführungsform überein, so dass zur Vermeidung von Wiederholungen auf die vorstehende Beschreibung verwiesen wird. Abwandlungen gegenüber der in den Figuren 1 bis 7 gezeigten Ausführungsform weisen dasselbe Bezugszeichen mit einem Apostroph auf.

Das Leitungsmittel 260' der Vorrichtung 100' ist in Form vierer gleichmäßig verteilter formstabiler Rohre ausgestaltet (zwei der Rohre sind in der Zeichenebene, eines befindet sich hinter und eines vor der Zeichenebene) ausgestaltet, welche eine axiale Leitungsmittellänge 261' aufweisen, welche ungefähr der Hälfte der Beutellänge 310' entspricht. Die Rohre sind an einem dem Förderkolben 270 zugewandten Rohrende jeweils als Dorn ausgestaltet und fungieren somit als weitere Öffnungsmittel 500' zu den am Förderkolben 270' angeordneten Öffnungsmitteln 500', was das fluidleitende Öffnen der Beutel 300' zusätzlich erleichtert.

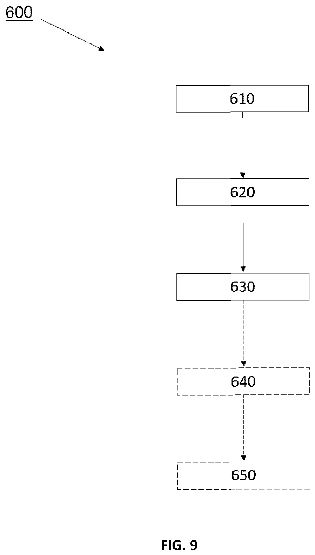

**Figur 9** zeigt ein Verfahren 600 zur Bereitstellung eines Knochenzementteigs 450 aus zwei Ausgangskomponenten mittels einer der Vorrichtungen 100, 100' gemäß den Figuren 1 bis 8 umfassend die Schritte 610, 620 und 630 sowie vorzugsweise auch die Schritten 640 und 650.

In einem Schritt 610 werden Austragskolben 250, 250' und der Förderkolben 2270, 270' zusammengeschoben und dabei deren Abstand zueinander verringert. Vorzugsweise wird dabei der Austragskolben 250, 250' in Richtung des Förderkolbens 270, 270' verbracht während der Förderkolben 270, 270' an seiner ursprünglichen Position innerhalb des Innenraums 210, 210' verbleibt. Auf diese Weise verbleibt im proximalen Teil 220, 220' des Innenraums 210, 210' mehr Raum zum späteren Anmischen des Knochenzementpulvers 400, 400' und der Monomerflüssigkeit 350, 350' unter Ausbildung des Knochenzementteigs 450.

Durch das Zusammenschieben der beiden Kolben 250, 250', 270, 270' wird der mindestens eine Beutel 300, 300' fluidleitend geöffnet und die Monomerflüssigkeit 350, 350' fließt in den distalen Teil 230, 230' des Innenraums 210, 210'.

In einem Schritt 620 werden der Austragskolben 250, 250' und der Förderkolben 270, 270' weiter zusammengeschoben, wobei weiterhin bevorzugt ist, dass sich dabei lediglich der Austragskolben 250, 250' bewegt, bis das Leitungsmittel 260, 260' am Förderkolben 270, 270' angeordnet ist, vorzugsweise mit dem Förderkolben 270, 270' in Kontakt steht. Das Leitungsmittel 260, 260' beabstandet in dieser Position die beiden Kolben 250, 250', 270, 270' voneinander und ein weiteres Zusammenschieben ist nicht möglich, ohne die strukturelle Integrität des Leitungsmittels 260, 260' zu zerstören. Indem das Leitungsmittel 260, 260' am Förderkolben 270, 270' angeordnet ist, kann die Monomerflüssigkeit 350, 350' direkt angrenzend am Förderkolben 270, 270' in das Leitungsmittel 260, 260' aufgenommen werden und durch das Leitungsmittel 260, 260' in den proximalen Teil 220, 220' des Innenraums 210, 210' gefördert werden. Beim Zusammenschieben der beiden Kolben 250, 250', 270, 270' wird der zumindest eine fluidleitend geöffnete Beutel 300a zusammengeschoben. Da das Leitungsmittel 260, 260' eine axiale Leitungsmittellänge 261, 261' aufweist, welche mindestens einem Viertel der Beutellänge 310, 310' entspricht, muss der Beutel 300, 300' im Wesentlichen nicht vollständig zusammengepresst werden, um die Monomerflüssigkeit 350, 350' im Wesentlichen vollständig in den proximalen Teil 220, 220' des Innenraums 210, 210' zu fördern. Dies erleichtert die Anwendung der Vorrichtung 100, 100' und erlaubt ein im Wesentlichen vollständiges Fördern der Monomerflüssigkeit 350, 350' in das Knochenzementpulver 400, 400'.

In einem Schritt 630 wird die Monomerflüssigkeit 350, 350' durch Anlegen eines Unterdrucks am Vakuumanschluss 550, 550' durch das Leitungsmittel 260, 260' hindurch in den proximalen Teil 220, 220' des Innenraums 210, 210' gefördert. Vorzugsweise wird der Unterdruck erst dann angelegt, wenn das Leitungsmittel 260, 260' am Förderkolben 270, 270' angeordnet ist.

Mit Beginn des Förderns der Monomerflüssigkeit 350, 350' startet die Ausbildung des Knochenzementteigs 450 aus den beiden Ausgangskomponenten. Um einen möglichst homogenen Knochenzementteig 450 zu erhalten, ist es bevorzugt, dass das Anmischen der Ausgangskomponenten aktiv durch den Anwender der Vorrichtung 100, 100' unterstützt wird. Dazu ist es bevorzugt, dass in einem optionalen Schritt 640 die Monomerflüssigkeit 350, 350' mit dem Knochenzementpulver 400, 400' mittels des Mischstabs 570, 570' bei angelegtem Unterdruck unter Ausbildung des Knochenzementteigs 450 vermischt wird. Der Mischstab 570, 570' ist bevorzugt mit dem Mischrohr 560, 560' lösbar verbunden, so dass eine axiale Bewegung des Mischstabs 570, 570' direkt auf das Mischrohr 560, 560' übertragen wird. Bevorzugt ist das Mischrohr 560, 560' mit einem Mischelement, vorzugsweise in Form einer Mischscheibe 575, 575' ausgestattet, um das Anmischen zu erleichtern. Durch das Anmischen bei angelegtem Unterdruck verringern sich Gaseinschlüsse im bereitgestellten Knochenzementteig 450.

Um den bereitgestellten Knochenzementteig 450 aus der Vorrichtung 100, 100' an eine gewünschte Stelle zu applizieren, ist es bevorzugt, dass in einem optionalen Schritt 650 der Mischstab 570, 570' vom Mischrohr 560, 560', vorzugsweise durch Entfernen des Griffs 580, 580', gelöst und aus dem Mischrohr 560, 560' gezogen wird. Bevorzugt wird vor oder nach dem Entfernen des Mischstabs 570, 570' aus dem Mischrohr 560, 560' das Mischrohr 560, 560' so weit aus dem Kartuschenkopf 540, 540' herausgezogen, dass das Mischrohr 560, 560' als Austragsschnorchel für den Knochenzementteig 450 verwendbar ist. Durch ein Vortreiben des Austragskolbens 250, 250' in Richtung des Kartuschenkopfs 540, 540' wird der Knochenzementteig 450 aus der Vorrichtung durch das Mischrohr 560, 560' ausgetragen. Bevorzugt wird das Vortreiben des Austragskolbens 250, 250' in Richtung des Kartuschenkopfs 540, 540' durch ein Vortreiben des Förderkolbens 270, 270' in Richtung des Kartuschenkopfs 540, 540', vorzugsweise unter Hilfe eines Austragshilfe, wie beispielsweise einer Austragspistole, ausgelöst. Dies erleichtert dem Anwender die Verwendung der Vorrichtung 100.

### Bezugszeichen

- 100, 100': Vorrichtung
- 200, 200': hohlzylinderförmige Kartusche
- 210, 210': Innenraum der Kartusche
- 220, 220': proximaler Teil des Innenraums
- 225, 225': proximales Innenraumende
- 230, 230': distaler Teil des Innenraums
- 250, 250': Austragskolben
- 260, 260': Leitungsmittel
- 261, 261': axiale Leitungsmittellänge
- 262: Kolbenabstand
- 263: Zylinderdurchführung
- 264: Hohlzylinderinnendurchmesser
- 265, 265': Porenscheibe
- 270, 270': Förderkolben
- 300, 300': Beutel
- 310, 310': Beutellänge
- 300a: geöffneter Beutel
- 350, 350': Monomerflüssigkeit
- 400, 400': Knochenzementpulver
- 450: Knochenzementteig
- 500, 500': Öffnungsmittel
- 540,540': Kartuschenkopf
- 545, 545': Kartuschenkopfdurchführung
- 546, 546': Schraubgewinde
- 550, 550': Vakuumanschluss
- 551, 551': Schlauch
- 560, 560': Mischrohr
- 570, 570': Mischstab
- 575, 575': Mischscheibe
- 580, 580': Griff
- 600: Verfahren zur Bereitstellung eines Knochenzementteigs
- 610: Zusammenschieben
- 620: fortgesetztes Zusammenschieben
- 630: Fördern
- 640: Mischen
- 650: Austragen

## Patentansprüche

1. Vorrichtung (100, 100') zum Bereitstellen eines Knochenzementteigs (450, 450') aus zwei Ausgangskomponenten, umfassend
eine hohlzylinderförmige Kartusche (200, 200') mit einem Innenraum (210, 210'), wobei in einem
proximalen Teil (220, 220') des Innenraums (210, 210') ein Knochenzementpulver (400, 400') als erste Ausgangskomponente und in einem distalen Teil (230, 230') des Innenraums (210, 210') mindestens ein Beutel (300, 300') enthaltend eine Monomerflüssigkeit (350, 350') als zweite Ausgangskomponente lagert,
wobei der Innenraum (210, 210') am proximalen Teil (220, 220') des Innenraums (210, 210') über einen Vakuumanschluss (550, 550') mit einer Unterdruckquelle verbindbar ist, wobei zwischen dem Knochenzementpulver (400, 400') und dem mindestens einen Beutel (300, 300') ein axial im Innenraum (210, 210') beweglicher Austragskolben (250, 250') und auf einer dem Austragskolben (250, 250') axial gegenüberliegenden Seite des mindestens einen Beutels (300, 300') ein axial im Innenraum (210, 210') beweglicher Förderkolben (270, 270') angeordnet ist, so dass bei einem Zusammenschieben von Austragskolben (250, 250') und Förderkolben (270, 270') der mindestens eine Beutel (300, 300') fluidleitend geöffnet werden und die Monomerflüssigkeit (350, 350') in den distalen Teil (230, 230') des Innenraums (210, 210') fließen kann,
wobei der proximale Teil (220, 220') und der distale Teil (230, 230') des Innenraums (210, 210') über ein Leitungsmittel (260, 260') fluidleitend miteinander verbunden sind,
**dadurch gekennzeichnet, dass**
sich das Leitungsmittel (260, 260') vom Austragskolben (250, 250') in den distalen Teil (230, 230') des Innenraums (210, 210') mit einer axialen Leitungsmittellänge (261, 261') erstreckt, welche mindestens einem Viertel einer Beutellänge (310, 310') des Beutels (300, 300') entspricht, so dass der Austragskolben (250, 250') und der Förderkolben (270, 270') beim Zusammenschieben durch das Leitungsmittel (260, 260') mit einem Kolbenabstand (262) beabstandet sind, welcher mindestens einem Viertel der Beutellänge (310, 310') entspricht.

2. Vorrichtung (100, 100') nach Anspruch 1, wobei das Leitungsmittel (260, 260') im Wesentlichen formstabil ist.

3. Vorrichtung (100) nach Anspruch 1 oder 2, wobei das Leitungsmittel (260) zumindest abschnittsweise als Hohlzylinder mit mindestens einer axial in einer Hohlzylinderwand verlaufenden Zylinderdurchführung (263) ausgeformt ist.

4. Vorrichtung (100) nach Anspruch 3, wobei der Hohlzylinder einen Hohlzylinderinnendurchmesser (264) aufweist, welcher eine zumindest teilweise Aufnahme des mindestens einen Beutels (300) erlaubt.

5. Vorrichtung (100') nach Anspruch 1 oder 2, wobei das Leitungsmittel (260') zumindest abschnittsweise als ein Rohr und/oder ein Schlauch ausgeformt ist.

6. Vorrichtung (100, 100') nach einem der vorhergehenden Ansprüche, wobei das Leitungsmittel (260, 260') einen fluidleitenden Leitungsmittelinnendurchmesser von 0,5 mm bis 2 mm aufweist.

7. Vorrichtung (100, 100') nach einem der vorhergehenden Ansprüche, wobei im distalen Teil (230, 230') des Innenraums (210, 210') mindestens ein Öffnungsmittel (500, 500') zum Öffnen des Beutels (300, 300') angeordnet ist.

8. Vorrichtung (100, 100') nach Anspruch 7, wobei das Öffnungsmittel (500, 500') als Dorn ausgeformt ist und/oder mindestens eine Schneidkante aufweist.

9. Vorrichtung (100, 100') nach einem der vorhergehenden Ansprüche, wobei ein dem Austragskolben (250, 250') axial gegenüberliegendes proximales Innenraumende (225, 225') des proximalen Teils (220, 220') des Innenraums (210, 210') mit einem Kartuschenkopf (540, 540') fluidleitend verschlossen ist.

10. Vorrichtung (100, 100') nach Anspruch 9, wobei der Vakuumanschluss (550, 550') am Kartuschenkopf (540, 540') angeordnet ist.

11. Vorrichtung (100, 100') nach Anspruch 9 oder 10, wobei in einer Kartuschenkopfdurchführung (545, 545') des Kartuschenkopfs (540, 540') ein axial im proximalen Teil (220, 200') des Innenraums (210, 210') verschiebbares Mischrohr (560, 560') angeordnet ist.

12. Vorrichtung (100, 100') nach Anspruch 11, wobei innerhalb des Mischrohrs (560, 560') ein lösbarer Mischstab (570, 570') angeordnet ist.

13. Verfahren (600) zum Bereitstellen eines Knochenzementteigs (450) aus zwei Ausgangskomponenten mittels einer Vorrichtung (100, 100') nach einem der vorhergehenden Ansprüche, umfassend die folgenden Schritte:
a. Zusammenschieben (610) von Austragskolben (250, 250') und Förderkolben (270, 270') unter Öffnen des mindestens einen Beutels (300, 300');
b. Fortgesetztes Zusammenschieben (620) von Förderkolben (270, 270') und Austragskolben (250, 250') bis das Leitungsmittel (300, 300') am Förderkolben (270, 270') angeordnet ist;
c. Fördern (630) der Monomerflüssigkeit (350, 350') durch das Leitungsmittels (260, 260') in den proximalen Teil (220, 200') des Innenraums (210, 210') durch Anlegen eines Unterdrucks am Vakuumanschluss (550, 550').

14. Verfahren (600) nach Anspruch 13 mittels einer Vorrichtung (100, 100') nach Anspruch 12, wobei in einem Schritt d. (640) das Knochenzementpulver (400, 400') und die Monomerflüssigkeit (350, 350') im proximalen Teil (220, 220') des Innenraums (210, 210') mittels des Mischstabs (570, 570') bei angelegtem Unterdruck unter Bereitstellung des Knochenzementteigs (450) vermischt werden.

15. Verfahren (600) nach Anspruch 14, wobei in einem Schritt e. (650) der Mischstab (570, 570') vom Mischrohr (560, 560') gelöst, der Mischstab (570, 570') aus dem Mischrohr (560, 560') gezogen und der angemischte Knochenzementteig (450) aus dem proximalen Teil (220, 220') des Innenraums (210, 210') durch das Mischrohr (560, 560') aus der Vorrichtung (100, 100') durch Vortreiben des Austragskolbens (250, 250') in Richtung des Kartuschenkopfs (540, 540') ausgetragen wird.

## Claims

1. A device (100, 100') for providing a bone cement paste (450, 450') consisting of two starting components, comprising
a hollow cylindrical cartridge (200, 200') having an interior (210, 210'), wherein a bone cement powder (400, 400') as the first starting component is stored in a proximal part (220, 220') of the interior (210, 210') and at least one pouch (300, 300') containing a monomer liquid (350, 350') as the second starting component is stored in a distal part (230, 230') of the interior (210, 210'), wherein the interior (210, 210') can be connected, at the proximal part (220, 220') of the interior (210, 210'), to a negative-pressure source via a vacuum connection (550, 550'), wherein a dispensing plunger (250, 250') which is axially movable in the interior (210, 210') is arranged between the bone cement powder (400, 400') and the at least one pouch (300, 300') and a delivery plunger (270, 270') which is axially movable in the interior (210, 210') is arranged on a side of the at least one pouch (300, 300') axially opposite the dispensing plunger (250, 250'), so that when the dispensing plunger (250, 250') and the delivery plunger (270, 270') are pushed together, the at least one pouch (300, 300') can be fluid-conductingly opened and the monomer liquid (350, 350') can flow into the distal part (230, 230') of the interior (210, 210'), wherein the proximal part (220, 220') and the distal part (230, 230') of the interior (210, 210') are fluidically connected to one another via a conduit means (260, 260'), **characterized in that**
the conduit means (260, 260') extends from the dispensing plunger (250, 250') into the distal part (230, 230') of the interior (210, 210') with an axial conduit means length (261, 261') which corresponds to at least a quarter of a pouch length (310, 310') of the pouch (300, 300'), so that the dispensing plunger (250, 250') and the delivery plunger (270, 270'), when pushed together by the conduit means (260, 260'), are spaced apart by a plunger distance (262) which corresponds to at least a quarter of the pouch length (310, 310').

2. The device (100, 100') according to claim 1, wherein the conduit means (260, 260') is substantially dimensionally stable.

3. The device (100) according to either claim 1 or claim 2, wherein the conduit means (260) is formed at least in portions as a hollow cylinder having at least one cylinder passage (263) extending axially in a hollow cylinder wall.

4. The device (100) according to claim 3, wherein the hollow cylinder has a hollow cylinder inner diameter (264) which allows at least partial accommodation of the at least one pouch (300).

5. The device (100') according to either claim 1 or claim 2, wherein the conduit means (260') is formed at least in portions as a pipe and/or a tube.

6. The device (100, 100') according to any of the preceding claims, wherein the conduit means (260, 260') has a fluid-conducting conduit means inner diameter of 0.5 mm to 2 mm.

7. The device (100, 100') according to any of the preceding claims, wherein at least one opening means (500, 500') for opening the pouch (300, 300') is arranged in the distal part (230, 230') of the interior (210, 210').

8. The device (100, 100') according to claim 7, wherein the opening means (500, 500') is formed as a mandrel and/or has at least one cutting edge.

9. The device (100, 100') according to any of the preceding claims, wherein a proximal interior end (225, 225') of the proximal part (220, 220') of the interior (210, 210') axially opposite the dispensing plunger (250, 250') is fluid-conductingly closed with a cartridge head (540, 540').

10. The device (100, 100') according to claim 9, wherein the vacuum connection (550, 550') is arranged on the cartridge head (540, 540').

11. The device (100, 100') according to either claim 9 or claim 10, wherein a mixing pipe (560, 560') which is axially displaceable in the proximal part (220, 200') of the interior (210, 210') is arranged in a cartridge head passage (545, 545') of the cartridge head (540, 540').

12. The device (100, 100') according to claim 11, wherein a detachable mixing rod (570, 570') is arranged inside the mixing pipe (560, 560').

13. A method (600) for providing a bone cement paste (450) consisting of two starting components by means of a device (100, 100') according to any of the preceding claims, comprising the following steps:
a. pushing together (610) the dispensing plunger (250, 250') and the delivery plunger (270, 270') to open the at least one pouch (300, 300');
b. continuing to push together (620) the delivery plunger (270, 270') and the dispensing plunger (250, 250') until the conduit means (300, 300') is arranged on the delivery plunger (270, 270');
c. delivering (630) the monomer liquid (350, 350') through the conduit means (260, 260') into the proximal part (220, 200') of the interior (210, 210') by applying a negative pressure to the vacuum connection (550, 550').

14. The method (600) according to claim 13 by means of a device (100, 100') according to claim 12, wherein in a step d. (640) the bone cement powder (400, 400') and the monomer liquid (350, 350') are mixed in the proximal part (220, 220') of the interior (210, 210') by means of the mixing rod (570, 570') under the application of negative pressure to provide the bone cement paste (450).

15. The method (600) according to claim 14, wherein in a step e. (650) the mixing rod (570, 570') is detached from the mixing pipe (560, 560'), the mixing rod (570, 570') is pulled out of the mixing pipe (560, 560') and the mixed bone cement paste (450) is dispensed from the proximal part (220, 220') of the interior (210, 210') through the mixing pipe (560, 560') out of the device (100, 100') by advancing the dispensing plunger (250, 250') in the direction of the cartridge head (540, 540').

## Revendications

1. Dispositif (100, 100') permettant de fournir une pâte de ciment osseux (450, 450') à partir de deux composants de départ, comprenant
une cartouche (200, 200') en forme de cylindre creux comportant un espace intérieur (210, 210'), dans lequel, dans une partie proximale (220, 220') de l'espace intérieur (210, 210'), une poudre de ciment osseux (400, 400') est stockée en tant que premier composant de départ et, dans une partie distale (230, 230') de l'espace intérieur (210, 210'), au moins une poche (300, 300') contenant un liquide monomère (350, 350') est stockée en tant que second composant de départ, dans lequel l'espace intérieur (210, 210') peut être relié à une source de dépression au niveau de la partie proximale (220, 220') de l'espace intérieur (210, 210') par l'intermédiaire d'un raccord à vide (550, 550'), dans lequel, entre la poudre de ciment osseux (400, 400') et l'au moins une poche (300, 300'), est disposé un piston de distribution (250, 250') mobile axialement dans l'espace intérieur (210, 210') et, sur un côté de l'au moins une poche (300, 300') opposé axialement au piston de distribution (250, 250'), est disposé un piston de transport (270, 270') mobile axialement dans l'espace intérieur (210, 210'), de sorte que, lorsque le piston de distribution (250, 250') et le piston de transport (270, 270') sont poussés l'un vers l'autre, l'au moins une poche (300, 300') peut être ouverte de manière conductrice de fluide et le liquide monomère (350, 350') peut s'écouler dans la partie distale (230, 230') de l'espace intérieur (210, 210'), dans lequel la partie proximale (220, 220') et la partie distale (230, 230') de l'espace intérieur (210, 210') sont reliées entre elles de manière conductrice de fluide par l'intermédiaire d'un moyen de conduite (260, 260'), **caractérisé en ce que** le moyen de conduite (260, 260') s'étend depuis le piston de distribution (250, 250') dans la partie distale (230, 230') de l'espace intérieur (210, 210') avec une longueur de moyen de conduite axiale (261, 261') qui correspond à au moins un quart d'une longueur de poche (310, 310') de la poche (300, 300'), de sorte que le piston de distribution (250, 250') et le piston de transport (270, 270') sont espacés, lorsqu'ils sont poussés l'un vers l'autre, par le moyen de conduite (260, 260') d'une distance entre les pistons (262) qui correspond à au moins un quart de la longueur de poche (310, 310').

2. Dispositif (100, 100') selon la revendication 1, dans lequel le moyen de conduite (260, 260') est sensiblement indéformable.

3. Dispositif (100) selon la revendication 1 ou 2, dans lequel le moyen de conduite (260) est formé au moins dans certaines sections comme un cylindre creux comportant au moins un passage de cylindre (263) s'étendant axialement dans une paroi de cylindre creux.

4. Dispositif (100) selon la revendication 3, dans lequel le cylindre creux présente un diamètre intérieur de cylindre creux (264) permettant une réception au moins partielle de l'au moins une poche (300).

5. Dispositif (100') selon la revendication 1 ou 2, dans lequel le moyen de conduite (260') est formé au moins dans certaines sections comme un tube et/ou un tuyau.

6. Dispositif (100, 100') selon l'une des revendications précédentes, dans lequel le moyen de conduite (260, 260') présente un diamètre intérieur de moyen de conduite conducteur de fluide allant de 0,5 mm à 2 mm.

7. Dispositif (100, 100') selon l'une des revendications précédentes, dans lequel au moins un moyen d'ouverture (500, 500') est disposé dans la partie distale (230, 230') de l'espace intérieur (210, 210') pour l'ouverture de la poche (300, 300').

8. Dispositif (100, 100') selon la revendication 7, dans lequel le moyen d'ouverture (500, 500') est formé comme un mandrin et/ou présente au moins une arête de coupe.

9. Dispositif (100, 100') selon l'une des revendications précédentes, dans lequel une extrémité d'espace intérieur proximale (225, 225') de la partie proximale (220, 220') de l'espace intérieur (210, 210'), laquelle extrémité d'espace intérieur proximale est opposée axialement au piston de distribution (250, 250'), est fermée de manière conductrice de fluide par une tête de cartouche (540, 540').

10. Dispositif (100, 100') selon la revendication 9, dans lequel le raccord à vide (550, 550') est disposé sur la tête de cartouche (540, 540').

11. Dispositif (100, 100') selon la revendication 9 ou 10, dans lequel un tube mélangeur (560, 560') pouvant coulisser axialement dans la partie proximale (220, 200') de l'espace intérieur (210, 210') est disposé dans un passage de tête de cartouche (545, 545') de la tête de cartouche (540, 540').

12. Dispositif (100, 100') selon la revendication 11, dans lequel une tige de mélange (570, 570') amovible est disposée à l'intérieur du tube mélangeur (560, 560').

13. Procédé (600) permettant de fournir une pâte de ciment osseux (450) à partir de deux composants de départ à l'aide d'un dispositif (100, 100') selon l'une des revendications précédentes, comprenant les étapes suivantes :
a. poussée l'un vers l'autre (610) du piston de distribution (250, 250') et du piston de transport (270, 270') pour ouvrir l'au moins une poche (300, 300') ;
b. poursuite de la poussée l'un vers l'autre (620) du piston de transport (270, 270') et du piston de distribution (250, 250') jusqu'à ce que le moyen de conduite (300, 300') soit disposé sur le piston de transport (270, 270') ;
c. transport (630) du liquide monomère (350, 350') à travers le moyen de conduite (260, 260') dans la partie proximale (220, 200') de l'espace intérieur (210, 210') en appliquant une dépression au niveau du raccord à vide (550, 550').

14. Procédé (600) selon la revendication 13 à l'aide d'un dispositif (100, 100') selon la revendication 12, dans lequel, au cours d'une étape d. (640), la poudre de ciment osseux (400, 400') et le liquide monomère (350, 350') sont mélangés dans la partie proximale (220, 220') de l'espace intérieur (210, 210') à l'aide de la tige de mélange (570, 570'), lors de l'application d'une dépression pour fournir la pâte de ciment osseux (450).

15. Procédé (600) selon la revendication 14, dans lequel, dans une étape e. (650), la tige de mélange (570, 570') est détachée du tube mélangeur (560, 560'), la tige de mélange (570, 570') est retirée du tube mélangeur (560, 560') et la pâte de ciment osseux (450) mélangée est distribuée à partir de la partie proximale (220, 220') de l'espace intérieur (210, 210') hors du dispositif (100, 100') par le tube mélangeur (560, 560') en faisant avancer le piston de distribution (250, 250') en direction de la tête de cartouche (540, 540').
